# EUROPEAN PATENT APPLICATION

(11) **EP 4 553 140 A1**
(43) Date of publication of application: **14.05.2025**
(21) Application number: 23834991.4
(22) Date of filing: 04.05.2023
(51) Int. Cl.: C12G 1/00, A01P 1/00

(54) **TREATMENT METHOD USING PLASMA-ACTIVATED WATER FOR TREATING AUXILIARY FOREST MATERIALS FOR WINE PRESERVATION, AND USE OF THE PLASMA-ACTIVATED WATER**

(30) Priority: 06.07.2022 ES 202230615
(71) Applicant: Universidad De La Rioja, 26006 Logroño (La Rioja) (ES)
(72) Inventor: SAINZ GARCÍA, Elisa, 26006 Logroño (La Rioja) (ES); GONZÁLEZ ARENZANA, Lucia, 26006 Logroño (La Rioja) (ES); SAINZ GARCÍA, Ana, 26006 Logroño (La Rioja) (ES); LÓPEZ ALFARO, Isabel, 26006 Logroño (La Rioja) (ES); MÚGICA VIDAL, Rodolfo, 26006 Logroño (La Rioja) (ES); GUTIÉRREZ VIGUERA, Ana Rosa, 26006 Logroño (La Rioja) (ES); MURO FRAGUAS, Ignacio, 26006 Logroño (La Rioja) (ES); ESCRIBANO VIANA, Rocio, 26006 Logroño (La Rioja) (ES); GONZÁLEZ MARCOS, Ana, 26006 Logroño (La Rioja) (ES); ALBA ELÍAS, Fernando, 26006 Logroño (La Rioja) (ES)
(74) Representative: Maslanka Kubik, Dorota Irena
(86) International application number: PCT/ES2023/070283
(87) International publication number: WO 2024/008983

(57) **Abstract**

The present invention relates to a treatment method using plasma-activated water (PAW) for treating auxiliary forest materials for wine preservation, such as bottle corks and wooden barrels. The method disinfects and/or decontaminates the materials by means of continuous contact between said materials and the PAW. The invention also relates to the use of the PAW to disinfect and/or decontaminate auxiliary forest materials used in wine production and preservation.

## Description

### FIELD OF THE INVENTION

The present invention relates generally to the field of wine production, and more specifically to a treatment method for disinfecting and/or decontaminating forest materials used in wine preservation for the purpose of preserving wine quality and food safety.

### BACKGROUND OF THE INVENTION

The wine industry is one of the most important agri-food sectors in Spain and Southern Europe. According to current statistics (OIV Statistical Report, 2018, www.oiv.int), Spain produces about 44 million hectoliters of wine, occupying third place after Italy and France. Furthermore, Spain has the largest vineyard surface area in the world (13% in total). The constant need for innovation in the wine sector to meet consumers' demands has generated strong competition on the market.

Moreover, the growing demand for natural products means that the food industry, and therefore the wine industry, is facing the challenge of providing safe, healthy, and minimally processed foods.

The organoleptic evolution of wines is highly favored when oak barrels are used for wine production and aging. Increasingly demanding consumers expect balanced wines with aromatic complexity. This is achieved with barrel aging since a series of wine/wood exchanges which enrich the aromas and mouthfeels of the wine occur, favoring micro-oxygenation, leading to a physically and chemically stable wine. Although a used barrel losses potential with respect to a new one, it is perfectly valid for use in the production of wine and alcoholic products at a lower cost. Therefore, proper maintenance of barrels is essential for reusing them. Microbiological or chemical contaminations are the main problems that arise during the aging of wine which may be altered or even become unsuitable for consumption when an undesirable flora develops (first 8 mm of the wood in contact with the wine). This is aggravated by reusing poorly maintained barrels, being able to give rise to the appearance of "acetic spoilage", phenolated nature or "Brett", "lactic spoilage", "ropiness", or "bitterness disease". A. Palacios et al., Enólogos. 77 (2012) 46-54.

Moreover, during aging, formed tartrate deposits adhere to the inner walls of the barrel, serving as a refuge for potentially contaminating microbial strains that may even block its porous structure. It has been established that a considerable volume of wine, of about 5 liters, is retained in the first millimeters of the staves in a 225-liter barrel. Furthermore, the deep penetration of microorganisms is favored by the microporous structure of the wood. All this complicates the barrel cleaning and disinfection tasks. This is particularly critical in the case of contamination by the yeast *Brettanomyces* or "Brett", which causes the phenolated nature of wine, with typical descriptors such as plastic, burnt rubber, barnyard, horse sweat, etc. This sensory problem is a topic that is widely discussed today and particularly affects aged wines or wines spending a long time in a barrel. A. Palacios et al., Enólogos. 77 (2012) 46-54.

In terms of the corks used to stopper wine bottles, they have the tendency to form TCA (2,4,6-trichloroanisole), which is commonly known as cork disease and affects 4% of bottled wines worldwide. TCA is a molecule formed by means of a chemical process due to the presence of chlorophenols. Chlorophenols are present in the environment, with cork oak and forest floors being some of the places where they can be found most frequently. For this reason, corks are the main source of TCA through which wine acquires negative characteristics; however, this process can be triggered in different steps of wine production due to the fact that chlorophenols are furthermore part of the wet environments characteristic of wineries. This problem is the most relevant in wine industry since, once wine acquires the organoleptic characteristics typical of TCA, it is impossible to eliminate them, so a rejection response by consumers occurs.

In 2017, Laithwaite's Wine conducted a study in which it was observed that 624 million bottles of wine were wasted in Great Britain yearly. The study mentioned different causes such as the consumers lacking knowledge about wine preservation or organoleptic wine defects. In this sense, it has been observed that the main cause of the reduction of the organoleptic characteristics of wine is related to unpleasant odors in the cork. These odors have different origins, but the most widespread is cork contamination by chlorinated compounds, particularly trichloroanisole (TCA), which is responsible for almost 80% of these problems.

Moreover, the wine industry is a fundamental client of the cork industry. The loss of market share of cork stoppers in comparison with alternative closures implies the need to reassess the cork industry which transmits, through its product, the intrinsic benefits of being environmentally friendly and socially responsible. The Iberian Peninsula dominates the world cork production and Spain represents 30% (506,000 Ha of cork oak forests). One of the challenges in the improvement in the cork industry is the reduction in the incidence of organochlorinated compounds which contribute to unpleasant aromas in wine. The interest in eliminating this organoleptic deviation and its causes is justified by the economic impact that it represents for the cork sector and the wine industry. Up until now, the application of water at high temperatures or the use of chemical preservatives have not been effective enough to eliminate the problem.

To solve the abovementioned problems, several methods of treating forest products related to wine production and preservation, for the purpose of maintenance and continuous reuse, are known in the art. One of the most widely used practices in wineries for disinfecting barrels is conventional sulfur wicking, which was used back in Roman times. This method consists of the burning of a sulfur pellet inside an empty barrel, the combustion of which produces sulfur dioxide, a compound with biocidal effects on wood. This practice helps to keep the stock of barrels of a winery free from any microorganism that may alter the wine.

However, Directive 98/8/EC2 of the European Commission prohibits the use of sulfur dioxide for barrel disinfection tasks. This directive has led to the urgent search for new disinfection solutions which allow this task to be carried out in a viable manner from an economic and operative viewpoint. In Spain there is a moratorium agreement on the prohibition of using sulfur wicking until 2025.

This scenario has led to the emergence of new alternative technologies for disinfecting barrels (by heat, ozone, cavitation, or sandblasting). However, none of them was capable of suitably responding to the needs of the sector and are potentially contaminating in different aspects.

Disinfection is not the same as sterilization (complete germ elimination), rather it involves the destruction of viable germs in order to greatly reduce residual populations by means of a chemical and/or physical action. In this sense, two categories relating to the new alternative barrel disinfection methods (alternative to sulfur wicking) can be established: by chemical or physical routes.

Chemical methods: these methods are performed by means of acidifying agents such as sulfur dioxide, used in liquid form on the wine in a sodium metabisulfite solution and in gas form on the wood and the wine, by means of sulfur combustion (the most widely used method discussed above) or the use of liquefied gas. Hydrogen peroxide, peracetic acid, potassium permanganate, and ozone (O₃) can also be used. In relation to ozone, it should be noted that it is a highly oxidative, toxic, and explosive gas. The use of ozone is only possible after the dissolution thereof in cold water to produce an active ozone solution containing between 3 and 5 mg/l of O₃. Ozone is a disinfectant, but it does not have any cleaning properties. Taking into account its mode of action, it is essential to use it on perfectly clean surfaces to enable disinfection. In the case of containers made of wood, it reacts partially with respect to the ozone, which greatly limits its action.

Physical methods: the most widely used physical method is the thermal method. In this case, mobile hot water generators, which can produce water at 80-90°C at between 80 and 210 bars, are used. Use at very high temperatures is unrealistic given that in such case the flow rates are very low. A pressure of 100-120 bars is sufficiently wood friendly. This technology allows detergent-free cleaning, although its disinfecting efficacy is limited to the surface of the wood. The temperature of its deeper layers (transmitted by water vapor at 105°C) increases very slowly since wood has a very low thermal conductivity. For this technology to be effective, treatment times must be very long. This is a highly limiting aspect given that, as already mentioned above, microorganisms can penetrate more than 8 mm into the wood. According to the foregoing and taking into account the different surfaces which may be in contact with the wine during its production, it can be considered that thermal treatments may be effective on steel or glass surfaces (with virtually no roughness), but not on surfaces of forest materials such as, for example, wood and cork (with very high roughness and porosity).

Another possible method is electromagnetic microwave treatment, which allows heating wood by exciting the water contained in the material. The selection of wavelength allows heating the wood from the center of the staves with low energy, while the shape and arrangement of the magnetron and the rotation of the barrel allow a homogenous irradiation within the metallic Faraday box that reflects the waves. Moreover, during ultrasound application a sonotron is introduced in the barrel filled with recycled water at 60°C, and the ultrasound (150 kHz) produces high pressures at a microscopic level (>2000 bars) on the surface of the wood and in the first few millimeters by water cavitation, removing compounds that impregnate the wood and destroying microorganisms while causing disinfection at the same time. Other less common physical methods include projecting dry ice (efficient and quick, but also costly and unable to ensure disinfection at the depth of the staves) and using negative oxygen.

All these methods have been studied scientifically and subjected to industrial testing, but none of them was able to improve the disinfection results obtained by means of sulfur wicking. Moreover, the cost of installation and processing time of some of these technologies (for example, ultrasound and microwave) is certainly prohibitive.

Another known disinfection method is cold atmospheric plasma. Although a study on its direct application on wooden products such as barrels has been conducted, it has never been applied at the industrial level. Plasma is the fourth state of matter. It is composed of positive and negative ions, electrons, excited and neutral atoms, free radicals, molecules in fundamental and excited states, and UV photons. Plasma can be classified into thermal (hot) plasma and non-thermal (cold) plasma according to the thermodynamic equilibrium of the temperature of its constituents. The temperature of cold plasma never exceeds a temperature of 100°C. Some of the non-thermal plasma sources widely used for food applications are dielectric-barrier discharges (DBD), plasma jets, and corona discharges. In recent years, many scientists and researchers have used cold plasma in various food applications: microbial disinfection, enzyme inactivation, improvement of the cooking quality of rice varieties, starch modification, improvement of seed germination, etc. (see, for example, R. Thirumdas et al., "Cold Plasma: A novel Non-Thermal Technology for Food Processing", Food Biophys. 10 (2014) 1-11).

Most studies on the antimicrobial activity of plasma have been conducted by applying plasma directly on foods or surfaces containing same. However, there are cases in which the direct application of a plasma source is not technically feasible or industrially profitable. This is the case, for example, of the plasma treatment of the inside of a barrel, in which, in order to be able to introduce the plasma equipment into the barrel, at least one of the fronts thereof must be disassembled. This is practically unviable in a winery.

A study on the use of plasma-activated water (PAW) as an disinfecting agent, instead of the direct application of cold or hot plasma, has commenced recently. As will be described in detail herein below, PAW contains reactive nitrogen and oxygen species with renowned antimicrobial activity that are generated when the plasma is contacted with water (primary reactive species) or from the subsequent interaction of said primary reactive species in secondary reactions (secondary reactive species).

For example, documents CN212456771U, RU2746976C1, and ES2314000T3 disclose devices for the production of PAW. However, none of these documents teaches or suggests the use of PAW produced for disinfecting the surfaces of forest materials.

There are very few publications on the use of PAW for decontaminating surfaces. Joshi et al ("Characterization of Microbial Inactivation Using Plasma-Activated Water and Plasma-Activated Acidified Buffer", J. Food Prot. 81 (2018) 1472-1480) study the use of PAW for disinfecting glass slides inoculated with *Enterobacter aerogenes.* Kamgang-Youbi et al ("Microbial decontamination of stainless steel and polyethylene surfaces using GlidArc plasma activated water without chemical additives", J. Chem. Technol. Biotechnol. 93 (2018) 2544-2551) confirmed the effectiveness of PAW for the surface decontamination of AISI304 stainless steel and high-density PET contaminated with *Saccharomyces cerevisiae*, *Staphylococcus epidermidis*, *Leuconostoc mesenteroides*, and *Hafnia alvei.* However, there is no known study which has satisfactorily applied PAW for disinfecting and decontaminating rough and porous materials, and more specifically forest materials such as wood and cork.

Moreover, documents ES2423255B1, ES2268459T3, ES2402890T3, ES2019562A6, ES2726598B2, ES2247180T3, for example, disclose various methods of treating cork products, such as bottle stoppers, for example, for reducing TCA contents. However, all these methods are based on the use of high temperatures, high pressures, long treatment periods, and/or the use of continuously rotating containers. However, high pressures and temperatures can cause adverse effects, such as irreversible distortions in stoppers, as indicated, for example, in document ES2423255B1. In turn, long treatment periods and rotating the containers (as well as the use of high pressures and temperatures also) cause increased implementation costs, and therefore a reduced profitability of the method.

Therefore, it would be desirable to provide an alternative method for the disinfection and/or decontamination treatment of auxiliary materials and products for wine production and preservation, and more specifically of forest products, such as wood and cork. Specifically, it would be desirable to provide a method that is an alternative to sulfur wicking, which produces results that are at least the same as or better than sulfur wicking, but which overcomes its drawbacks, specifically preventing the toxic problems derived from the use of sulfur dioxide. Likewise, it would be desirable for said method not to cause any adverse effects on the treated materials (corks and wood), such as irreversible deformations, while at the same time reducing the economic costs associated with its implementation in comparison with alternatives known in the state of the art as mentioned above.

### SUMMARY OF THE INVENTION

To overcome the problems mentioned above, the present invention proposes a new treatment method for treating auxiliary forest materials used for wine preservation in order to disinfect and/or decontaminate them for the purpose of preserving wine quality and food safety. Specifically, the method is based on contacting the material to be disinfected and/or decontaminated with plasma-activated water (PAW).

The present invention also proposes the use of plasma-activated water (PAW) to disinfect and/or decontaminate auxiliary forest materials used in wine production and preservation.

The attached dependent claims describe preferred embodiments of the treatment method and of the use of PAW of the present invention.

### BRIEF DESCRIPTION OF THE FIGURES

The present invention will be better understood in reference to the following drawings which illustrate a preferred embodiment of the invention, provided by way of example, and should not be interpreted as limiting of the invention in any way.
Figure 1 schematically shows a system for producing plasma-activated water for use thereof in a method according to the preferred embodiment of the present invention.
Figure 2 schematically depicts the steps of disinfecting a wooden barrel according to several preferred embodiments of the present invention.
Figure 3 shows the chromatograms of PAW samples treated during different time periods, in which the phenol reaction products related to each of the reactive species in the PAW samples can be seen.
Figure 4 shows the superposition of the peak of the phenol reaction product of the hydroxyl radical OH* from the chromatograms of four PAW samples.
Figure 5 is a bar graph depicting the log growth of viable *Brettanomyces* cells per gram of wood from contaminated staves treated with distilled water (DW) and with various PAW samples according to the present invention. The different letters indicate statistically significant differences (p < 0.05).
Figure 6 is a bar graph depicting the mean concentration of TCA (ng/l) of artificially contaminated corks treated with distilled water (DW) and with various PAW samples according to the present invention. The different letters indican statistically significant differences (p < 0.05).

### DETAILED DESCRIPTION OF PREFERRED EMBODIMENTS

As mentioned above, the present invention is based on the use of plasma-activated water (PAW) for the disinfecting and/or decontaminating treatment of auxiliary forest materials intended for wine production and preservation.

PAW technology has a number of advantages over current disinfection and/or decontamination methods. First, it is a low-cost technology as it only consumes electricity and compressed air for the generation of plasma in many cases and does not require chemicals, filters, or other consumable materials. Furthermore, it is generated at atmospheric pressure and room temperature, so no auxiliary installations are required. Moreover, it can be applied with the current barrel washing systems (pressurized water lances), so barrels can be cleaned and sterilized at the same time, saving water, energy, and process time. Lastly, it is an environmentally friendly technology since no toxic or waste chemicals are produced.

However, PAW technology is an emerging technology that has not yet been studied extensively or applied at an industrial level. Specifically, no known publication mentions the use of PAW for disinfecting surfaces made of wood or other forest materials.

Plasma-activated liquids are generated when contacting (directly or indirectly) a plasma source with a liquid. This interactions causes the generation and/or transfer of reactive chemical species to the liquid. Plasma-activated solutions can be used as disinfecting solutions to be applied in food industries. The most extensively studied plasma-activated liquid is plasma-activated water (PAW). Various publications have demonstrated its antibacterial effects (see, for example, M.J. Traylor et al., "Long-term antibacterial efficacy of air plasma-activated water", J. Phys. D. Appl. Phys. 44 (2011) 472001).

PAW has a composition and physicochemical properties different from those of water. It usually presents an acidic pH, changes in redox potential and conductivity, and the presence of reactive oxygen species (ROS) and reactive nitrogen species (RNS) (A. Mai-Prochnow et al., "Microbial decontamination of chicken using atmospheric plasma bubbles", Plasma Process. Polym. (2020)).

The antimicrobial capacity of PAW is produced by means of several processes: [a] gaseous phase: formation of reactive species in gaseous phase due to the interaction of charged particles (electrons, neutrons, etc.) and ultraviolet radiation with plasma gas and the surrounding atmosphere; [b] gas-liquid phase: dilution in water of the reactive chemical species generated in the gaseous phase or those generated due to plasma-liquid interaction, particularly those having a relatively long lifetime, such as ozone, atomic oxygen, or nitric oxide, which act as precursors for other ROS and RNS, such as hydrogen peroxide, nitrates, or nitrites; and [c] liquid phase: secondary reactions of long-lived reactive species arising, for example, due to the instability of nitrites in an acidic medium. These cyclic reactions justify the presence, for days, of short-lived highly cytotoxic reactive species, for example, hydroxyl radicals (OH*), acidified nitrites (NO*, NO₂*), and peroxynitrites (O=NOOH).

Some of the most biocidal chemical species of PAW (OH*, NO*, NO₂*, and O=NOOH) are generated once the plasma source has been turned off (SLS, "short-lived" reactive species). SLSs can be produced during the generation of PAW (when plasma is contacted with the atmosphere and water), but they have such a short life (in the order of milliseconds) that their presence in PAW cannot be due to their dilution in water. SLSs that are finally found in PAW are generated from the reactions of "long-lived" reactive species (hydrogen peroxide, nitrates, and nitrites) produced in PAW once the plasma source has been turned off. The SLSs resulting from these secondary reactions are "transitory" reactive species that have highly cytotoxic properties and cause a prolonged antimicrobial activity of PAW even several days after exposing the water to plasma discharge.

The main secondary reactions arise because nitrites are not stable in acidic conditions (pH < 3.5). Diagrams of the main secondary reactions taking place in PAW are shown below. Nitrous acid (HNO₂), which is in acid-base equilibrium with nitrites [a], breaks down in acidic conditions to yield nitric oxide (NO*) radical and nitrogen dioxide (NO₂*) radical through reaction [b]. Nitrogen dioxide (NO₂*) radical furthermore undergoes hydrolysis in an aqueous medium to produce nitrite (NO₂⁻) ion as the end product through reaction [c]. NO* and NO₂* can also react with dissolved oxygen to produce nitrite (NO₂⁻) and nitrate (NO₃⁻) ions according to general reactions [d] and [e], respectively. NO* and NO₂* nitrogen radicals formed in these secondary reactions have strong cytotoxic properties and are probably one of the main causes of the cytotoxic effects of nitrites in acidic conditions. For this reason, they are referred to as "acidified nitrites". Furthermore, in acidic conditions, the reaction of nitrites (NO₂⁻) with hydrogen peroxide (H₂O₂) can generate peroxynitrites (O=NOOH) [f]. Peroxynitrites can react directly with microorganisms or indirectly by means of breaking down into OH* and NO₂* [g]. This is one of the pathways for the generation of hydroxyl (OH*) radicals.

NO₂⁻ + H⁺ ↔ HNO₂ [a]

2 HNO₂ → NO* + NO₂* + H₂O [b]

2 NO₂* + H₂O → NO₃⁻ + NO₂⁻ + 2 H⁺ [c]

4 NO* + O₂ + 2 H₂O → 4 NO₂⁻ + 4 H⁺ [d]

4 NO₂* + O₂ + 2 H₂O → 4 NO₃⁻ + 4 H⁺ [e]

NO₂⁻ + H₂O₂ + H⁺ → O=NOOH + H₂O [f]

O=NOOH → OH* + NO₂* [g]

The formation of acidified nitrites (NO* and NO₂*) and OH* radicals by means of secondary reactions are key to the biocidal properties of PAW that are prolonged in time.

### Hydroxyl (OH*) radical

OH* radicals are probably the most important reactive species produced by the plasma treatment of aqueous solutions. They can non-selectively oxidize most organic compounds with which they come into contact and are the main source of hydrogen peroxide in plasma systems by radical recombination. In relation to their biocidal capacity, the part most affected by OH* radicals is the outer cell wall of microorganisms, including the cell membrane. The cell membrane, made up mostly of organic compounds such as lipids, proteins, and polysaccharides, is susceptible to attack by OH* radicals. Lipids are the cell membrane macromolecules most vulnerable to oxidation. The reactions of lipids with OH* radicals occur due to the removal of H from unsaturated carbon bonds in fatty acids which, in the presence of oxygen, cause lipid peroxidation. Similarly, OH* radicals can damage membrane proteins by means of the abstraction of H from the α carbon of peptide bonds -CO-NH- between the amino acids of a chain attached to peptides. Attack by OH* radicals leads to peroxidation and excision of the protein backbone. The combined effect of these impairments leads to cell death.

### NO* and NO₂* radicals ("acidified nitrites")

Acidified nitrites have a significant antimicrobial effect against a wide range of pathogenic organisms, including viruses (such as SARS-CoV-1, SARS-CoV-2), bacteria, and fungi. Some of the damage they cause in microorganisms are: membrane protein oxidation, reaction with metalloenzymes, which leads to the consumption of available iron, metabolic enzyme inactivation, DNA impairment due to oxidative damage, lipid peroxidation which damage cell membranes, etc. This multifactorial damage results in severe dysfunctions and, ultimately, cell death.

As mentioned above, the present invention relates to a treatment method using plasma-activated water (PAW) for treating auxiliary forest materials, used in wine preservation, to disinfect and/or decontaminate said materials. Specifically, the forest materials which are subjected to disinfection and/or decontamination can be selected from the group consisting of bottle corks and wooden containers, such as wooden barrels.

In the case of corks, the main purpose of the method according to the present invention is to cause a reduction of the anisoles present therein, more specifically of TCA (2,4,6-trichloroanisole). Said TCA reduction is preferably at least 50%, more preferably at least 75%, with respect to the amount of TCA in the corks before treatment.

In the case of wooden containers, specifically wooden barrels, the main purpose of the method according to the present invention is to reduce the amount of yeast, specifically *Brettanomyces* yeast, therein with respect to the amount of *Brettanomyces* in the containers before treatment. Said *Brettanomyces* reduction is preferably at least 1 log, more preferably at least 3 log, with respect to the *Brettanomyces* content of the containers before treatment.

More specifically, the treatment method according to the present invention comprises the steps of:
- optionally, producing PAW by means of applying plasma to starting water,
- optionally, cleaning the material to be disinfected by means of pressurized water, and
- immediately after the step of cleaning, if it is performed, continuously contacting the material to be disinfected and/or decontaminated with PAW for a time period sufficient to obtain a desired disinfection and/or decontamination level of the material to be disinfected and/or decontaminated, preferably for a minimum time period of 3 hours.

The present invention also relates to the use of plasma-activated water (PAW) to disinfect and/or decontaminate auxiliary forest materials used in wine production and preservation.

As mentioned above, the reactive species causing the disinfecting and/or decontaminating effect in PAW may be present during a relatively prolonged period, for example, of several days. Therefore, the step of producing PAW can be performed as part of the method according to the present invention, can be performed as an independent method in the same location, or can even be acquired from an external commercial supplier, depending on the requirements and technical limitations of the installation in which the method herein disclosed is implemented.

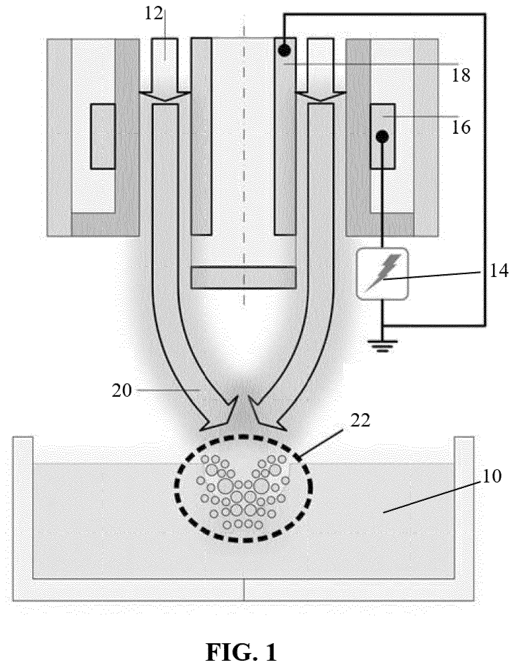

According to the preferred embodiment of the present invention, to generate PAW useful for use thereof in the treatment method herein disclosed, a system for producing cold atmospheric plasma-activated water, as schematically depicted in Figure 1, is used.

The bottom part shows a tank (10) comprising distilled water used as starting water that will be plasma-activated. Distilled water is useful to perform the tests described herein below since it does not have the biocidal effect of chlorine that is present in tap water. However, according to an additional preferred embodiment of the present invention, tap water is used as starting water for the production of PAW.

Air (12) is used as gas for plasma production since it is the gas which produces the most OH* radicals, and it is also the most inexpensive gas.

The system further consists of a power generator (14) and two electrodes (16, 18). When air (12) passes through the system, a plasma flow (20) which is projected onto the water tank (10) is produced, giving rise to plasma-water (22) interactions that produce the reactive species by means of the chemical reactions described above.

The parameters used for the generation of PAW according to the preferred embodiment of the present invention are as follows:
Plasma gas: air
Plasma gas (air) flow: 60 slm
Plasma power: 500 W
Volume of treated water: 2 liters of distilled water
Processing time: between 1.5 and 30 minutes

Table 1 below shows the nomenclature of the four PAW samples produced by means of the tests herein described and the time during which the starting water was subjected to treatment with plasma for the production of each of said samples:

**Table 1**

| PAW | Generation time (minutes) |
|---|---|
| PAW-1.5 | 1.5 |
| PAW-5 | 5 |
| PAW-15 | 15 |
| PAW-30 | 30 |

By means of the tests that will be described herein below, it has been determined that the physicochemical characteristics of PAW which significantly reduce the load of *Brettanomyces* are: pH < 4.5, EC (electrical conductivity) > 50 µS/cm, ORP (oxidation-reduction potential) > 350 mV, NO₃⁻ > 3 mg/l and NO₂⁻ > 0.5 mg/l.

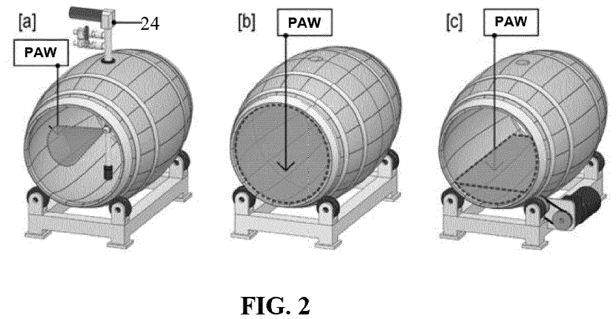

Once the PAW has been produced, it is then used in the disinfecting/decontaminating treatment method of the present invention. However, before performing this step of disinfecting/decontaminating by means of contacting with PAW, the surface of the material to be disinfected/decontaminated should be cleaned, particularly in the case where the treatment method is to be applied on wooden containers, such as wooden barrels. Cleaning the inside of the barrel aims to eliminate encrusted material and sediments. This is a common practice in wineries and, at present, is performed before sulfur wicking. This washing is usually performed with high-pressure water using lances (24) (see Figure 2a). According to an additional preferred embodiment of the present invention, the pressurized water used in the step of cleaning is also PAW.

The step of continuously contacting the inside of the barrel with PAW is then performed. Preferably, this step of contacting with PAW (disinfection) is performed immediately after the step of cleaning. PAW will be introduced therein using the same hole through which the washing water was introduced. To that end, the same washing lance (24) can be used.

For PAW to eliminate *Brettanomyces* from the barrel (as indicated above, *Brettanomyces* on the inner walls of the barrel can be up to 8 mm deep), PAW must be in contact with the entire inner surface of the barrel for a sufficient time (preferably for a time of at least 3 hours). To that end, there are two alternatives. According to a preferred embodiment, as shown in Figure 2b, the entire volume of the barrel is filled with PAW. According to another preferred alternative embodiment, shown in Figure 2c, a minimum volume of PAW is introduced in the barrel and the barrel is rotated continuously, such that the PAW comes into contact with the entire inner surface thereof. In this case, the consumption of PAW is significantly reduced in comparison with the embodiment shown in Figure 2b. These systems for rotating the barrels are common in wineries and used to empty the contents of said barrels.

In the case of applying the treatment method according to the present invention for reducing TCA in corks, the step of continuously contacting the corks with PAW is performed by immersing the corks completely in PAW in a suitable container. It is not required to perform any type of shaking or movement of the corks, rather the treatment method is completely static.

In any case, the method according to the present invention is preferably performed at room temperature and atmospheric pressure.

### EXAMPLES

Several examples of the application of the treatment method herein disclosed are described below.

### 1) Analysis for quantification of the reactive species present in PAW:

First, a test was carried out to quantify the reactive species present in each of the PAW samples prepared as described above.

The high reactivity of the most biocidal secondary species present in PAW (OH*, NO*, NO₂*, and O=NOOH) means that their detection and quantification, particularly in liquid phase, must be performed indirectly. The main methods for detecting these species use chemicals that react selectively with these radicals to generate relatively stable products that can be detected spectroscopically by electron paramagnetic resonance, fluorimetry, or high-performance liquid chromatography (HPLC).

In this case, for the indirect detection and quantification of the reactive species present in PAW, the method described by Lukes et al ("Aqueous-phase chemistry and bactericidal effects from an air discharge plasma in contact with water: evidence for the formation of peroxynitrite through a pseudo-second-order post-discharge reaction of H2O2 and HNO2", Plasma Sources Sci. Technol. 23 (2014) 015019) was taken as a reference. This method is based on the reaction between phenol (C₆H₅-OH) and OH*, NO*, and NO₂* radicals. A phenol solution in water (≈ 2 x 10⁻² M) is prepared, from which 5 ml are taken for mixing with 95 ml of PAW, and then the mixture is heated at 50°C for 24 hours. The solution is then filtered with a 0.45 µm filter disk and subjected to high-performance liquid chromatography (HPLC): 20 µl; column C18; mobile phase of 1.0 ml/min using the following elution gradient:

| t, min | Acetic acid: 1 % (V/V) | Acetonitrile |
|---|---|---|
| 0 | 90 | 10 |
| 7 | 60 | 40 |
| 13 | 90 | 10 |

Total time: 16 min; pressure: ≈ 90 bars at 90/10, ≈ 60 bars at 60/40; DAD detector at 260 nm (reference: 699 nm).
The reaction products are: benzoquinone (phenol + OH*), 4-nitrosophenol (phenol + NO*) and 2-nitrophenol (phenol + NO₂*).
The reaction times are: hydroquinone: ≈ 5 minutes, 4-nitrosophenol : ≈ 5.4 minutes, phenol : ≈ 8.2 minutes, and 2-nitrophenol : ≈ 11.2 minutes.

For the quantification of reaction products (µg/l), the corresponding calibration curves were prepared from identifying the absorbance values of reaction product solutions of known concentrations. Benzoquinone and 2-nitrophenol standards with a purity ≥ 98 % were obtained from Sigma-Aldrich (Madrid, Spain), and 4-nitrosophenol standard with a purity ≥ 98 % was obtained from TCI (Tokyo, Japan). Methanol (≥ 99.5 %) was acquired from Scharlab (Sentmenat, Barcelona, Spain).

Individual stock solutions were prepared at a concentration of 10⁻² M in water-methanol (95-5) and stored in glass bottles at 8°C.

Calibration curves were prepared by dilution with deionized water with the following concentrations: benzoquinone 1.35x10⁻⁵ M, 2-nitrophenol 1.60x10⁻⁴ M, and 4-nitrosophenol 2.54x10⁻⁴ M.

The calibration curves were as follows:

| Compound | Equation | *R* |
|---|---|---|
| Benzoquinone | *A* (mAU)= 0.09 + 6.99×10⁵ *c* (M) | 0.9996 |
| 2-Nitrophenol | *A* (mAU)= -0.24 + 1.50×10⁵ *c* (M) | 0.9997 |
| 4-Nitrosophenol | *A* (mAU)= -1.00 + 3.78×10⁵ c (M) | 0.9997 |

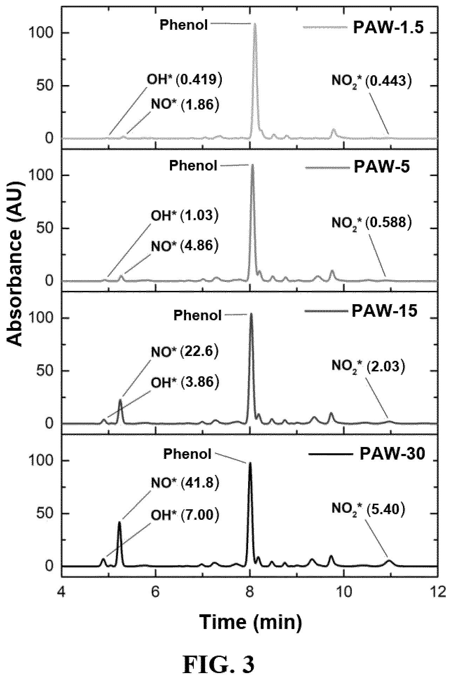

Chromatograms were obtained for the various PAW samples object of study (PAW-1.5, PAW-5, PAW-15, and PAW-30) in which there were identified signals relating to the phenol reaction products of the most important reactive species in relation to the disinfecting/decontaminating capacity of PAW:hydroxyl radicals (OH*; benzoquinone), acidified nitrites (NO*; 4-nitrosophenol and NO2*; 2-nitrophenol), and phenol itself (Figure 3).

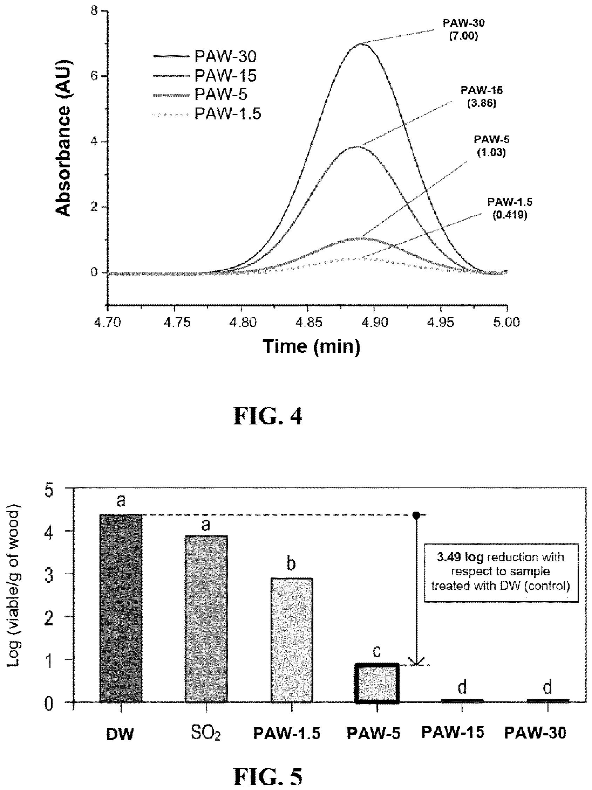

Due to the importance of hydroxyl radicals for both applications (Brett disinfection in wooden barrels and TCA reduction in corks), chromatograms relating to the hydroxyl radical for all the PAW samples considered are identified with greater detail in Figure 4.

Finally, each of the reactive species present was indirectly quantified (from the phenol reaction products) by means of the method described above, and the results are shown in Table 2.

**Table 2. Concentration (µg/l) of phenol reaction products**

| PAW | Concentration (µg/l) of phenol reaction product | | |
|---|---|---|---|
| | OH* (benzoquinone) | NO* (4-nitrosophenol) | NO₂* (2-nitrophenol) |
| PAW-1.5 | 8.2 | 227.0 | 209.5 |
| PAW-5 | 23.1 | 465.1 | 253.9 |
| PAW-15 | 91.7 | 1873.0 | 696.1 |
| PAW-30 | 168.1 | 3404.8 | 1729.6 |

### 2) Tests relating to the barrel disinfection method:

### Test materials

Wooden oak barrels used in aged wine production were used. The barrels were naturally contaminated with *Brettanomyces.* The barrels object of study were dismantled and stave portions measuring 5 x 5 cm were obtained. Mean *Brettanomyces* contamination of a sample configured by 4 stave portions was determined to be 4.35 ± 0.26 logarithms of viable cells per gram of wood.

### Treatments applied

Sulfur wicking (current reference treatment): 3 contaminated staves were introduced (5 x 5 cm) in a 4-liter capacity glass bottle. A 5 gram sulfur pellet was hung and ignited with a lighter, thereby giving off SO₂ vapors. The bottle was hermetically closed with its cover and a parafilm and kept that way for 30 minutes. This method reproduces to scale the same conditions for the application of this technology performed today.

### Immersion with PAW:

Three naturally contaminated staves were introduced in respective containers with 250 ml of PAW considered in the study. The face corresponding to the inside of the barrel was completely submerged up to 3 cm (microorganisms penetrate up to 8 mm into the wood). Five treatments were considered per immersion: 1 with distilled water (DW, control) and 4 with PAW (PAW-1.5, PAW-5, PAW-15, and PAW-30).

### Tests performed

Analysis of the microbial inactivation of PAW:
Contaminated staves were marked 10 mm from the toasted surface. This is the height at which all the *Brettanomyces* which contaminates the wood is expected to be found. The staves were placed independently in plastic containers (one container per stave) with the toasted surface (the surface corresponding to the inside of the barrel and where *Brettanomyces* is found) facing down. Then, 250 ml of PAW (each of the 4 being considered) and distilled water (forming control replicates) were added. Each sample consisted of 3 replicates.

Once the treatment time (3-hour immersion in all cases) has elapsed, the staves were extracted and planed up to 10 mm deep. Chips were collected in sterile plastic bags that had been weighed previously.

Once the wood chips have been collected, each of the bags containing same was weighed to know the weight of chips collected from each sample. Each bag was then placed, open, inside a sterile storage bottle and 600 ml of warm TSB (tryptic soy broth, recovery medium) were added, ensuring that the chip was well soaked. The bottles with the bags were subjected to orbital shaking for 24 hours at 28°C and 80-100 rpm. After said infusion time, the bottles with the chips were removed from the orbital shaker, respecting the order in which they were placed for shaking. The infused TSB was then extracted from each sample in the laminar follow cabinet under aseptic conditions. The infused culture medium was sieved through a sterile net curtain and placed in sterile centrifuge bottles. The samples were then centrifuged at 4°C and 10000 g for 30 minutes. After centrifugation, the supernatant was immediately removed and the sediment was resuspended in Ringer's solution (under cooling to maintain cell viability for up to 7 days), making up to a volume of 15 ml in sterile plastic tubes.

Finally, the samples were analyzed by first performing treatment corresponding with PMA (propidium monoazide) and DNA was extracted from the samples. Once DNA has been extracted, quantitative PCR was performed with EvaGreen, expressing the results of the population of viable *Brettanomyces* in genomic units per gram of wood (see Figure 5).

### Results obtained

As can be seen in Figure 5, treatment with PAW-5 obtains a 3.49 log reduction with respect to the control sample (treated with distilled water, DW). Treatment with PAW-1.5 (obtained with 1.5 minutes of starting water treatment with plasma) obtains only a 1.46 log reduction which is, however, still better than the reference treatment by means of sulfur wicking. Treatments with PAW-15 and PAW-30 achieve a total reduction.

According to a preferred embodiment of the present invention, treatment with PAW-5 was considered optimum since it obtains a significant reduction of *Brettanomyces* (3.49 log). Although the reduction is not a total reduction, as in the case of PAW-15 and PAW-30, the energy consumption with respect to PAW-15 is 3 times lower (3 times less treatment time for PAW production). However, the choice of any of the PAW disclosed herein, all of which are within the scope of protection of the attached claims, will depend on preferences and technical restrictions of the specific application in which the treatment method herein disclosed will be applied.

### 3) Tests relating to the method for reducing TCA (2,4,6-trichloroanisole) in corks

### Test materials

100% natural cork stoppers (for 750 ml wine bottles) were used. These corks did not present TCA contamination. Some of the corks of the series used were analyzed to rule out the presence of polyhaloanisoles and polyhalophenols. The result was negative in all cases.

Four uncontaminated corks were submerged in respective 95 gram glass containers with 80 ml of a solution prepared with 400 ng/l of TCA. The containers were placed in a rotary shaker at 50 rpm for 5 hours. Once treatment has ended, each cork was analyzed individually. The mean TCA concentration of the 4 artificially contaminated corks was 26.59 ± 1.64 ng/l.

### Treatments applied for reducing TCA (2,4,6-trichloroanisole)

Five treatments were considered per immersion: 1 with distilled water (DW, control) and 4 with PAW (PAW-1.5, PAW-5, PAW-15, and PAW-30). The same PAW as those used for disinfecting wooden barrels were used. For each treatment, 3 artificially contaminated corks as described above were used.

The corks were (individually) submerged (in triplicate) in 95 gram glass containers with 80 ml of each type of solution (DW and 4 PAW) for 3 hours. No movement, rotation, or shaking of any type was used. It should be noted that the method of applying the different solutions (DW and 4 PAW) was the same as that used for disinfecting the wooden barrels: 3 hours of immersion.

### Tests performed

Polyhaloanisole and polyhalophenol analysis: all the corks of the study (artificially contaminated corks treated with distilled water and with PAW) were analyzed following the chemical analysis methods OIV-MA-AS315-16 (Determination of releasable 2,4,6-trichloroanisole in wine by cork stoppers, Resolution OIV/OENO 296/2009) and OIV-MA-AS315-17 (Determining the presence and content of polychlorophenols and polychloroanisoles in wines, cork stoppers, wood, and bentonites used as atmospheric traps, Resolution OIV/OENO 374/2009) corresponding to the "Compendium of International Methods of Wine and Must Analysis" of the "International Organization of Vine and Wine" (OIV). These methods simulate the migration phenomena of 2,4,6-trichloroanisole (TCA), 2,4,6-trichloroanisole, 2,4,6-trichlorophenol, 2,3,4,6-tetrachloroanisole, 2,3,4,6-tetrachlorophenol, pentachloroanisole, and pentachlorophenol that can take place between a cork stopper and bottled wine. Cork stoppers are macerated in a water-alcohol solution until equilibrium is obtained. Polyhaloanisoles and polyhalophenols are extracted from the headspace of an aliquot part of the macerate by means of the solid-phase microextraction (SPME) technique and analyzed by means of gaseous phase chromatography, with detection by mass spectrometry (GC/MS).

### Results obtained

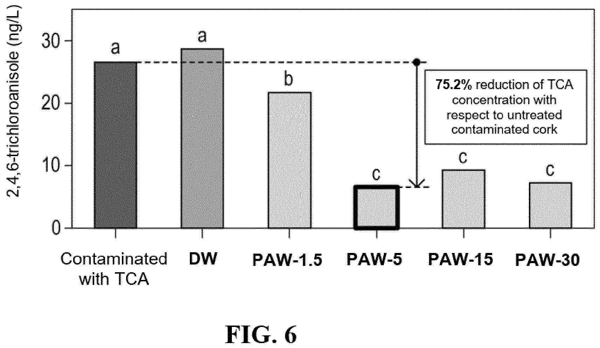

The decontamination results are shown in Figure 6. As can be seen, treatment with PAW-5 causes a 75.2% reduction of the TCA contained in untreated contaminated corks (control sample). Treatment with distilled water (DW) is in no case effective. This suggests that artificial contamination with TCA is not superficial (as occurs with naturally contaminated corks) since distilled water has not "entrained" TCA from the surface.

Assuming that distilled water and PAW have reached the inner pores of the cork, all the TCA which is reduced comes from the decomposition obtained through hydroxyl (OH*) radicals of PAW.

Treatment with PAW-1.5 (obtained with 1.5 minutes of generation) obtains an 18.1% reduction of the TCA contained in untreated contaminated corks. Treatments with PAW-15 and PAW-30 achieve a reduction similar to that of PAW-5.

Based on these results, according to a preferred embodiment of the present invention, treatment with PAW-5 was considered optimum for TCA reduction in corks, since it obtains a significant TCA reduction (more than 75%). Furthermore, energy consumption with respect to PAW-15 (which furthermore removes a similar amount of TCA) is 3 times lower (3 times less treatment time for PAW production).

As can be seen from the preceding tests, in both applications considered herein (Brett disinfection in wood and TCA reduction in corks), the preferred treatment is the same: static immersion (of contaminated wood and corks) for 3 hours using PAW-5. The plasma-activated water subjected to a 5-minute activation treatment (PAW-5) is characterized by an OH* concentration of more than 23 µg/l (23.1 µg/l as shown in Table 2 above). However, in other applications, the use of PAW-1.5 (also included in the scope of protection defined by the attached claims) can also be considered as desirable depending on technical limitations and other specific aspects of the installation in which the method of the present invention is applied. PAW-1.5 (water subjected to 1.5-minute plasma activation treatment) is characterized by an OH* concentration of more than 8 µg/l (specifically 8.2 µg/l as shown in Table 2 above).

Similarly, it has been considered that the preferred physicochemical characteristics of the plasma-activated water (PAW) to disinfect and/or decontaminate auxiliary forest materials used in wine production and preservation are as follows: a pH of less than 4.5, an EC of more than 50 µS/cm, an ORP of more than 350 mV, an NO₃⁻ concentration of more than 3 mg/l, an NO₂⁻ concentration of more than 0.5 mg/l, and an OH* concentration of more than 8 µg/l, preferably more than 23 µg/l. Said parameters are already achieved with a 1.5-minute plasma activation treatment (PAW-1.5). The most preferred plasma activation treatment time is 5 minutes (PAW-5).

Techniques widely known in the art were used to determine the pH value, the EC (electrical conductivity) value, the ORP (oxidation-reduction potential) value, and NO₃⁻ and NO₂⁻ concentrations.

Although the present invention has been described herein in reference to preferred embodiments thereof, it should be understood that these embodiments are explanatory and non-limiting. One skilled in the art will be able to readily implement modifications and variations to the embodiments herein described without, however, departing from the scope of protection defined exclusively by the attached claims.

## Claims

1. A treatment method using plasma-activated water (PAW) for treating auxiliary forest materials for wine preservation for the disinfection and/or decontamination of said materials, which comprises contacting the material to be disinfected and/or decontaminated with PAW.

2. The method according to claim 1, **characterized in that** the step of contacting the material to be disinfected and/or decontaminated with PAW is performed continuously for at least 3 hours.

3. The method according to any of the preceding claims, **characterized in that** an anisole reduction is obtained in the material subjected to decontamination with PAW.

4. The method according to claim 3, **characterized in that** a TCA (2,4,6-trichloroanisole) reduction is obtained.

5. The method according to claim 4, **characterized in that** a TCA reduction of at least 50% is obtained.

6. The method according to claim 5, **characterized in that** a TCA reduction of at least 75% is obtained.

7. The method according to any of claims 3 to 6, **characterized in that** the material which is subjected to decontamination by means of contact with PAW consists of wine bottle corks.

8. The method according to any of claims 1 to 2, **characterized in that** it comprises a prior step of cleaning the material to be disinfected and/or decontaminated by means of pressurized water immediately before the step of continuously contacting with PAW.

9. The method according to claim 8, **characterized in that** the pressurized water used in the step of cleaning is PAW.

10. The method according to any of claims 1, 2, 8, or 9, **characterized in that** it is applied to wooden containers and a *Brettanomyces* reduction is obtained.

11. The method according to claim 10, **characterized in that** a *Brettanomyces* reduction of at least 1 log is obtained with respect to the wooden container before treatment.

12. The method according to claim 11, **characterized in that** a *Brettanomyces* reduction of at least 3 log is obtained with respect to the wooden container before treatment.

13. The method according to any of claims 10 to 12, **characterized in that** it is applied to wooden barrels.

14. The method according to claim 13, **characterized in that** the step of continuously contacting the wooden barrel with PAW is performed by filling the entire volume of the barrel with PAW.

15. The method according to claim 13, **characterized in that** the step of continuously contacting the wooden barrel with PAW is performed by introducing a minimum volume of PAW in the barrel and rotating the barrel continuously such that the PAW comes into contact with the entire inner surface thereof.

16. The method according to any of the preceding claims, **characterized in that** it comprises the prior step of producing PAW by means of applying plasma on starting water, using air as plasma gas, a plasma gas flow of 60 slm, a plasma power of 500 W.

17. The method according to any of the preceding claims, **characterized in that** it comprises the prior step of producing PAW by means of applying plasma on starting water, wherein the time of treating the starting water with plasma is between 1.5 minutes and 30 minutes.

18. The method according to claim 16 or 17, **characterized in that** the time of treating the starting water with plasma is 5 minutes.

19. The method according to any of claims 16 to 18, **characterized in that** the starting water is distilled water.

20. The method according to any of claims 16 to 18, **characterized in that** the starting water is tap water.

21. The method according to any of the preceding claims, **characterized in that** the PAW used has a pH of less than 4.5, an EC of more than 50 µS/cm, an ORP of more than 350 mV.

22. The method according to any of the preceding claims, **characterized in that** the PAW used comprises an NO₃⁻ concentration of more than 3 mg/l and an NO₂⁻ concentration of more than 0.5 mg/l.

23. The method according to any of the preceding claims, **characterized in that** the PAW used comprises an OH* concentration of more than 8 µg/l.

24. The method according to claim 23, **characterized in that** the PAW used comprises an OH* concentration of more than 23 µg/l.

25. Use of plasma-activated water (PAW) to disinfect and/or decontaminate auxiliary forest materials used in wine production and preservation.

26. Use of plasma-activated water (PAW) according to claim 25, wherein the PAW used has a pH of less than 4.5, an EC of more than 50 µS/cm, an ORP of more than 350 mV.

27. Use of plasma-activated water (PAW) according to claim 25 or 26, wherein the PAW used comprises an NO₃⁻ concentration of more than 3 mg/l and an NO₂⁻ concentration of more than 0.5 mg/l.

28. Use of plasma-activated water (PAW) according to any of claims 25 or 27, wherein the PAW used comprises an OH* concentration of more than 8 µg/l, preferably more than 23 µg/l.
